(19) **Europäisches Patentamt European Patent Office Office européen des brevets**

(11) **EP 1 555 936 B1**

(12) **EUROPEAN PATENT SPECIFICATION**

(45) Date of publication and mention of the grant of the patent:
**27.05.2009 Bulletin 2009/22**

(21) Application number: **03707281.6**

(22) Date of filing: **11.03.2003**

(51) Int Cl.:
*A61B 5/042* (2006.01)    *A61N 1/05* (2006.01)

(86) International application number:
**PCT/SE2003/000403**

(87) International publication number:
**WO 2004/006762 (22.01.2004 Gazette 2004/04)**

(54) **MEDICAL SYSTEM WITH A MULTI-DOT ELECTRODE, WHERE THE SUB-SIGNALS ARE COMBINED INTO A SYTHETIC REFERENCE**

MEDIZINISCHES SYSTEM MIT EINER MEHRPUNKT-ELEKTRODE, BEI DEM DIE UNTERSIGNALE IN EINER SYNTHETISCHEN REFERENZ KOMBINIERT SIND

SYSTEME MEDICAL DOTE D'UNE ELECTRODE MULTI-POINT, LES SOUS-SIGNAUX ETANT COMBINES EN UNE REFERENCE SYNTHETIQUE

(84) Designated Contracting States:
**CH DE FR IE IT LI**

(30) Priority: **10.07.2002 SE 0202186**

(43) Date of publication of application:
**27.07.2005 Bulletin 2005/30**

(73) Proprietor: **St Jude Medical AB**
**175 84 Järfälla (SE)**

(72) Inventors:
• **STRANDBERG, Hans**
**S-172 65 Sundbyberg (SE)**
• **LINDEGREN, Ulf**
**S-112 64 Stockholm (SE)**

(56) References cited:
**EP-A1- 1 013 303          DE-A1- 4 211 852**
**US-A- 4 848 352          US-A- 6 064 905**
**US-B1- 6 357 447          US-B1- 6 418 348**

**Description**

Field of the invention

The present invention relates to a system according to the preamble of the independent claim.

Background of the invention

Electrode bodies adapted for sensing and/or stimulating electrical activities of a heart and provided with a plurality of electrically insulated electrode surfaces at small distances from one another are previously known through e.g. US-6,064,905 and US-4,848,352.

In US-6,064,905 a multi-element tip electrode mapping catheter is known comprising a tip section including a multi-element tip electrode mounted at the distal end of the tip section comprising a plurality of electrode members electrically isolated from one another and arranged such that, during use of the catheter within the heart, at least two different electrode members are capable of contacting the endocardium tissue at one time.

US-4,848,352 relates to a method for cardiac pacing and sensing using combination of electrodes where a cardiac pacing lead is inserted into a heart chamber. The lead carries a plurality of separate electrodes positioned at the distal tip of the lead and electrically isolated from each other. A separate electrical conductor is provided for each electrode. The electrodes may e.g. be entered in an electrical conductive relation in order to allow sensing to take place from any or all of the electrodes.

Signals detected e.g. inside a heart by a conventional heart electrode where the signal is detected either between two electrodes arranged at a distance from each other and close to the distal end (by using a bipolar electrode) or between a single electrode inside the heart and an indifferent electrode arranged e.g. at the housing of the heart stimulator are often influenced by different kinds of interference. Normal interference signals may emanate from human body generated signals such as muscle interference or from unsuitable electromagnetic fields in the patient's environment.

The interference may be reduced by the use of a bipolar electrode system, where a short electrode distance is one reducing factor. This is a reason for using an electrode surface having several small dot electrodes.

However, experiments performed on animals have shown that the difference between the signals obtained from two dot electrodes at a multi-dot electrode unit is difficult to detect when using detectors conventionally used in heart stimulators. The reason is that the signals are too similar.

A conventional heart signal detector works on a pair of electrode surfaces and with traditional heart pacemaker signal processing. The traditional signal processing is amplification, band-pass filter and amplitude detection. The detector level relative the original signal amplitude is then most often between 0,5 mV up to 10 mV.

When sensing a heart signal with a lead comprising several small electrodes close to each other, e.g. in a multi-dot electrode unit, there may be a problem to detect the signal if conventional pacemaker signal processing is used because the differential signal may be only a small part of the total signal amplitude. This part of the amplitude may be in the order 0,1 to 1 mV and can often be less than 0,1 mV. Depending on the vector of the heart activity propagation in relation to the electrode surfaces, the differential signal can even be reduced to nearly zero. Due to the fact that the propagation vector of heart activity varies the differential signal amplitude thereby also varies.

One way to optimise signal detection would be to monitor all combinations of electrode pairs and momentarily select the pair with highest amplitude or at least avoid the pair with zero amplitude. As the propagation vector of heart activity continuously varies, the monitoring and optimisation process must be continuously ongoing which is considered difficult to implement.

The object of the present invention is to solve the above-stated problem regarding detection when using small electrodes arranged at a small distance from one another, e.g. when using a multi-dot electrode unit.

Summary of the invention

The above-mentioned object is achieved by the present invention according to the independent claim.

Preferred embodiments are set forth in the dependent claims.

Thus, the object is achieved by creating a synthetic reference signal from all or at least three of the dot electrodes to which the sensed signal from each of the electrodes may be related.

According to the present invention the amplitudes of all (or most of) the dot electrodes are combined to a synthetic reference signal. The only requirement for the signal processing is that there is some difference between any of the dot electrode pairs. As all dot electrodes are grouped together, external interference is totally avoided and high amplification for the differential signal may be used.

The processing means that processes the detected signals may be arranged either close to the multi-dot electrode unit (e.g. in the electrode lead tip) or e.g. in a heart stimulator to which the electrode lead is connected.

Thereby the signal between the synthetic reference signal and any electrode will have a significant amplitude, as the amplitude of the heart signal not can be totally cancelled. The requirement of the electrodes used to create the synthetic reference signal is that not two pairs of electrodes have the same vectors.

This results in a signal separated from zero between any of the electrodes and the synthetic reference signal, and that each signal in relation to the synthetic reference signal will have enough amplitude to contribute for detection and the cancellation phenomenon in bipolar systems, i.e. two dots, is thereby avoided.

The signal processing performed by the processing means is simple and straightforward. There is no need to filter the signals and the unsuitable side effects of the pacemaker filters (may be referred to as "memory-effects" of the filter) used today are avoided.

The present invention accomplishes total avoidance of some of the signal problems in pacemakers as signal detection of different types of interference such as external interference or far field heart signals.

In a preferred embodiment of the present invention an electrode lead is provided with the multi-dot electrode unit and a processing means arranged close to the multi-dot electrode unit and supplies detection signal(s) from the processing means through the lead into the heart stimulator.

Short description of the appended drawings

Figure 1 shows a block diagram of the medical system according to a preferred embodiment of the present invention.

Figure 2 shows a block diagram that schematically illustrates the multi-dot electrode unit and the processing means according to the preferred embodiment.

Figure 3 shows a diagram of a preferred embodiment as a schematic of electronics of the present invention.

Figure 4 is a cut through view of a multi-dot electrode unit in the distal tip of an electrode lead.

Figure 5 is a cross-sectional view of the multi-dot electrode unit in the distal tip of an electrode lead as shown in figure 4.

Figure 6 shows signal recordings from seven dot electrodes of a multi-dot electrode unit placed in the ventricle of an animal.

Figure 7 shows a small part of the signal recordings as in figure 6 in a larger scale.

Figure 8 shows differences of signal recordings of seven dot electrodes (S1-S7) of a multi-dot electrode unit where electrode S 1 is used as a reference.

Figure 9 shows differences of signal recordings of seven dot electrodes (S1-S7) of a multi-dot electrode unit where the synthetic reference signal (SR) calculated according to the present invention is used as a reference.

Figure 10 shows three signal tracings of different processed signals.

Figure 11 shows the same signals as in figure 10 but with another time scale.

Detailed description of preferred embodiments of the invention

A multi-dot electrode unit used in connection with the present invention is preferably arranged at the distal end of an electrode lead. The multi-dot electrode unit includes a plurality of dot electrodes electrically insulated from one another and arranged at small distances from one another. An exemplary distance between dot electrodes is less than 1 mm.

In its broadest aspect a dot electrode should be regarded as a small electrode surface having an optional geometric shape, e.g. circular, elliptical, rectangular, square, band-shaped etc. In case of a circular dot electrode the diameter is in the interval of 0,05 - 1 mm.

The distance between adjacent dot electrode surfaces is preferably less than 1 mm. The distance between the most distant dot electrodes in a multi-dot electrode unit is less than 10 mm.

The electrode lead provided with the multi-dot electrode unit will be able to pick up nearly the same electrical signal on each dot electrode.

In order to perform bipolar sensing using the dot electrodes the differential signal (that is the signal between two dot electrodes) is small and a special effort must be taken in the design to avoid so-called common mode effects.

Initially a general overview of the present invention will be given.

According to the present invention the signals from N dot electrodes are added together in order to create an unnormalized synthetic reference signal. N could be any number between 3 and the total number of dot electrodes. The total number of dot electrodes may be very large but a suitable number is between 10 and 30, preferably 20. In one embodiment N is in the range 4-8. The created signal is then divided by N and a normalized synthetic reference signal is obtained. The differential signal to be used for detection is made in relation to the normalized synthetic reference signal.

The following example further illustrates how the synthetic reference signal is created and how a differential signal for a specific dot electrode in relation to the created synthetic reference signal is determined.

The signal amplitude at a dot electrode "i" is designated $U_i$ in relation to a reference. This reference may be obtained from a point capable of providing a long-term stable reference level, e.g. a separate indifferent electrode at the pacemaker housing or the battery in the pacemaker. The un-normalized synthetic reference signal will then be:

$$\Sigma \ (U_1 + \ldots + U_N)$$

A normalized synthetic reference signal (SR-signal) will then be:

$$SR\text{-signal} = (1/N) \times \Sigma \ (U_1 + \ldots + U_N)$$

The differential signal $A_{diff(i)}$ for dot electrode i will then be:

$$A_{diff(i)} = U_i - (1/N) \times \Sigma \ (U_1 + \ldots + U_N)$$

This differential signal is amplified to a suitable detecting level. As the short distance between the dot electrodes essentially reduces the influence of interference the only filtering necessary to perform is to transfer the signal shape to a shape that is easy to detect.

To detect the heart signal from activity near the dot electrodes the contribution from all electrodes is most optimal. As the difference signal of each dot electrode relative the synthetic reference can be both positive and negative the difference signals must be processed prior the signals are combined.

According to a preferred embodiment of the present invention the absolute values of the difference signals are determined and then added together to form an indication signal. This sign conversion may be achieved by rectification. Alternatively and according to an alternative embodiment, the difference signals instead are squared and then added together to form the indication signal.

Still a further possibility is to form a value representing the signal contents for the difference signals upon which the indication signal is formed.

According to a preferred embodiment of the present invention the signal processing means is arranged close to the multi-dot electrode unit. As indicated above the multi-dot electrode unit preferably is placed at the distal tip of the electrode lead. However, any position along the electrode lead may be possible without departing from the scope of the present invention as it is defined in the appended claims.

Thus, according to this preferred embodiment the processing means includes a specific amplifier and detector circuit placed close to the electrode tip. In this case a fully integrated circuitry with enough small size to be placed within one or a few cubic millimetres is arranged in the electrode tip.

With regard to the power supply of the processing means two conductors are arranged in the electrode lead from the heart stimulator (that includes a battery) to the electrode tip. One of the conductors may be the one used for heart stimulation. The information from the signal detection may also be sent from the electrode tip to the heart stimulator on the same pair of conductors. By using switched loading an altered voltage level may transfer information in binary format from the electrode tip into the heart pacemaker. The signal transfer does not contain complete information with regard to full signal morphology but only detection information is transferred. The detection information may then be used by control means in the heart stimulator to control e.g. the pulse generation.

The signal processing performed by the processing means to detect heart activity may be done in several ways, for example band pass filtering of the signals, adding signals, correlate signals together etc. The type of electronics can be of several types, e.g. traditional analogue, analogue to digital converted and then digitally processed, analogue sampled and processed in analogue way by a so-called switch capacitor technique. Thus, the present invention may be implemented in many different ways where some will be described in the following.

The preferred implementation of the processing means in view of technical realisation as micro-power electronics with smallest dimensions to be assembled into the electrode tip is the so-called sampled switch cap technique.

However, for easier understanding of this implementation the description is as if it were traditional analogue technique keeping in mind that there is methods for direct translation between those techniques.

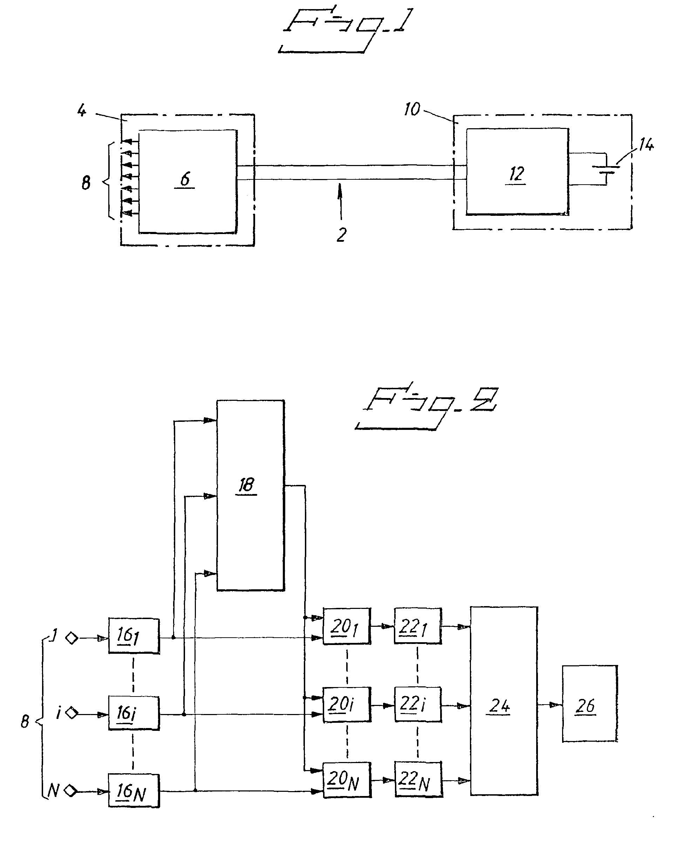

Figure 1 shows a block diagram of the medical system according to a preferred embodiment of the present invention. The system comprises an electrode lead 2 provided with a multi-dot electrode unit 4 including a processing means 6. The multi-dot electrode unit 4 is provided with a plurality of dot electrodes 8 and may be arranged near or in contact with a heart wall, e.g. in the right ventricle of the heart.

In the figure the multi-dot electrode unit is arranged close to the distal end of the electrode lead, i.e. in the electrode lead tip, but may also be arranged at another position along the lead, e.g. within the form of a cylindrical ring electrode at a predetermined distance from the distal end.

The electrode lead 2 is coupled to an implantable heart stimulator 10 that inter alia comprises heart therapy and control means 12 and power supply means 14.

Figure 2 shows a block diagram that schematically illustrates the multi-dot electrode unit and the processing means according to the preferred embodiment. Each of the dot electrodes $8_1$-$8_N$ is connected to a respective amplifying means $16_1$-$16_N$ in order to amplify the dot electrode signals sensed by the dot electrodes.

The signal ($U_1$ - $U_N$) provided to each amplifier 16 must be in relation to any reference voltage. The reference voltage could be any stable voltage potential such as the power supply voltage or a derivate of such voltage.

If the amplitude of a sensed dot electrode signal is in the order of 0,1 - 2 mV the amplification should be in the range of 50-2000 in order to obtain a dynamic detection voltage in the order of 1 Volt. The amplifying means are connected to a synthetic reference calculation means 18 where a synthetic reference signal (SR-signal) is calculated.

The synthetic reference signal and the amplified dot electrode signals are applied to difference signal means $20_1$-$20_N$ where differences between each of the amplified dot electrode signals and the synthetic reference signal are formed according to the formula $A_{diff(i)} = U_i$ - SR-signal, where i = 1 ... N.

According to a preferred embodiment of the present invention the difference signals are applied to rectifying means $22_1$-$22_N$ where the signals are rectified and these rectified signals are in turn applied to a summing means 24. The summing means determines an indication signal that is applied to a detector means 26 provided with predetermined heart event detection criteria that may include one or many threshold(s) representing different predetermined heart events to be detected. One or many detection signals is(are) generated by the detector means as a result of the these comparisons.

The multi-dot electrode unit is in particular suitable for detecting "fast" heart events in the close vicinity of the electrode. If the multi-dot electrode unit is placed in the atrium these fast heart events could be spontaneous or stimulated P-waves and atrial fibrillation or flutter and if the electrode is placed in the ventricle spontaneous or stimulated R-waves could be detected.

According to an alternative embodiment of the present invention the processing means is arranged at a distance from the multi-dot electrode unit, e.g. inside the body in an implantable heart stimulator or outside the body in an external medical device. In this alternative embodiment each dot electrode is connected via a separate conductor in the electrode lead to the processing means located e.g. inside a heart stimulator housing.

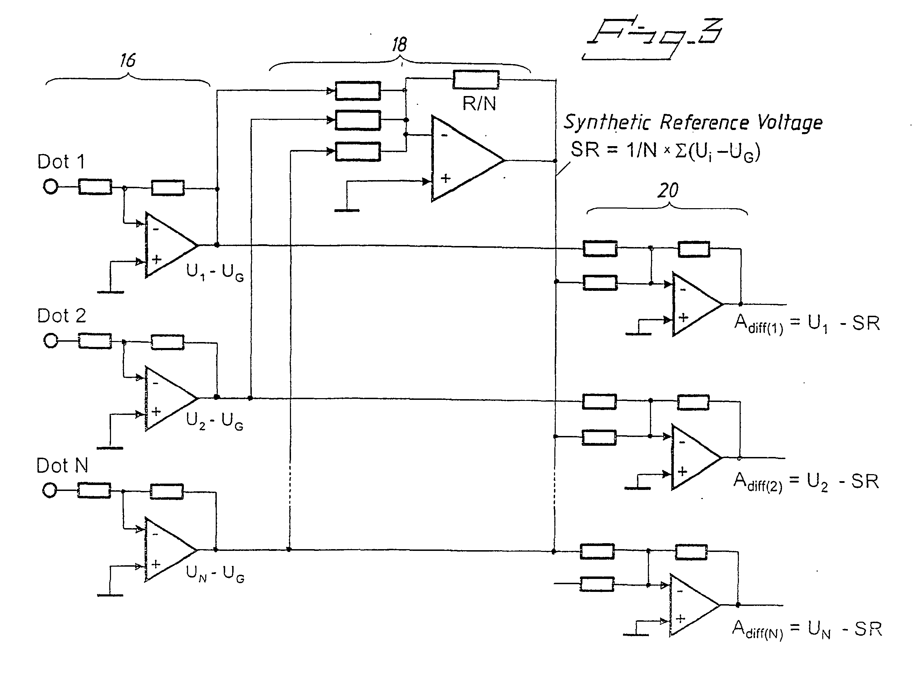

Figure 3 shows a diagram as a schematic of electronics illustrating the circuitry of the preferred embodiment of the present invention. In the illustrated embodiment three of the N dot electrodes are shown. The rectangles represent resistors having the resistance R except where indicated a resistor value of R/N and the triangles represent operational amplifiers.

$U_1$ - $U_N$ are the dot electrode potentials in relation to an electrical reference point.

In the figure $U_G$ represents a ground potential against which each of the dot electrode potentials are related to. It should be noted that $U_G$ is a conducting surface (e.g. a metal surface) close to the multi-dot electrodes (e.g. inside said processing means). This potential ($U_G$) is not stable compared to e.g. a dot electrode since it is related to the battery source and the housing of the implant. However, if this defined ground potential ($U_G$) or zero potential is used internally in the processing means as the earth potential then no additional disturbance or interference is induced during the further processing of the signals.

Using the same reference signs as in figure 2 the circuitry in figure 3 includes the amplifying means 16 that amplify the sensed heart signals and generate dot electrode signals ($U_1$-$U_N$), the synthetic reference calculation means 18 that calculates the synthetic reference signal (SR-signal) and the difference signal means 20 that determines the difference signals $A_{diff(1)}$ - $A_{diff(N)}$.·

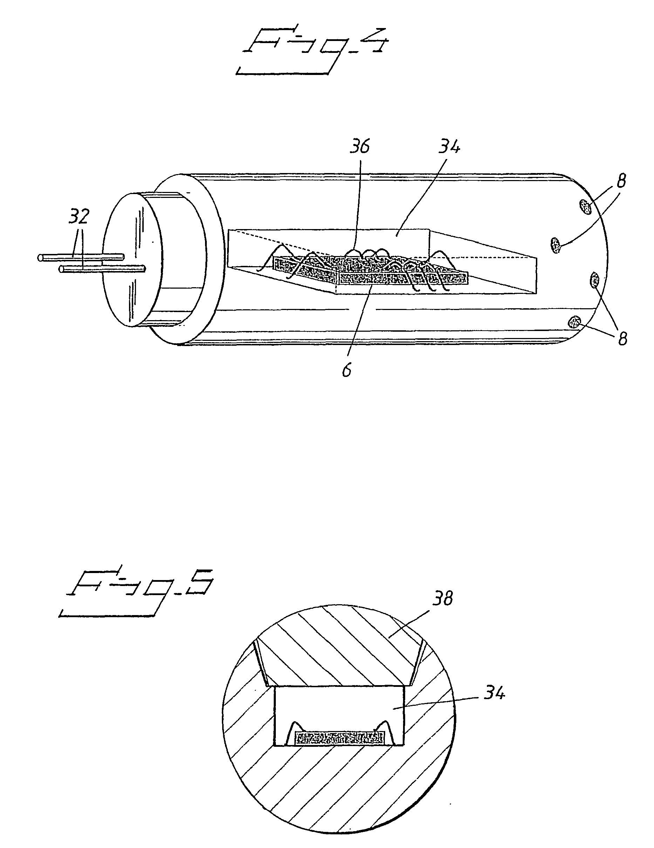

Figure 4 is a cut through view of a multi-dot electrode unit in the distal tip of an electrode lead with processing means 6 and two conductors 32 connecting the processing means to the control means in the pacemaker. The distal tip is a hermetic capsule of ceramic material with a cavity 34 housing the processing means. The diameter of the capsule is in the order of 3 mm. The two conductors pass through the wall of the capsule where it is connected to the processing means via wire bonding 36. The dot electrodes 8 are evenly spread on the end of the electrode tip and are connected to the processing means.

Figure 5 is a cross-sectional view of the multi-dot electrode unit in the distal tip of an electrode lead as shown in figure 4. The cavity 34 is closed with a lid 38 inserted from above and hermetically sealed to the capsule. The sealing may consist of a low temperature melting glass or some type of metallic soldering.

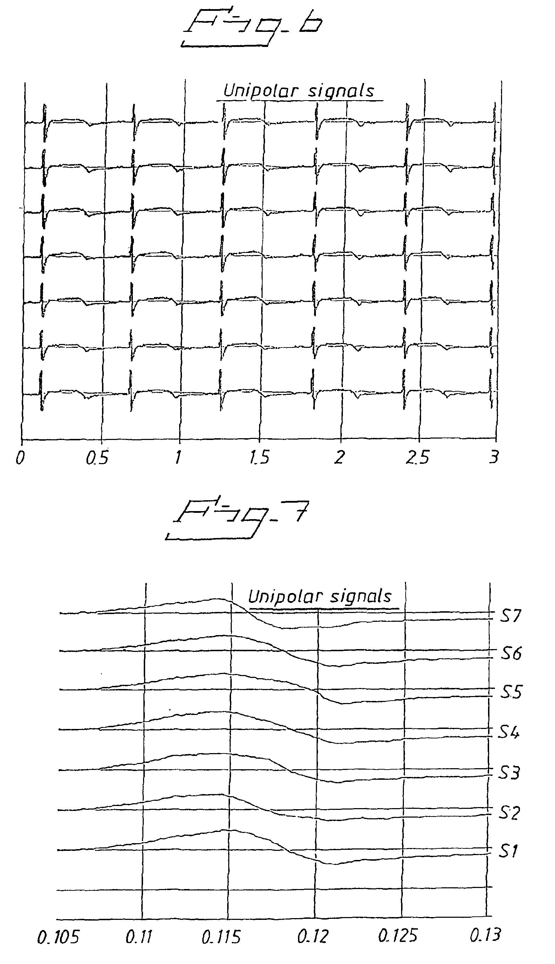

Figure 6 shows signal recordings from seven dot electrodes of a multi-dot electrode unit placed in the ventricle of an animal where the R-waves easily may be identified. The signals are unipolar which means that they are measured between each of the electrodes and an electrode surface at the capsule of the pacemaker. The time scale (horizontal axis) is from 0 to 3 seconds. The signal from electrode 7 is at the top and electrode 1 at the bottom. Only small non-obvious differences between the electrode signals may be identified.

Figure 7 shows a small part of the signal recordings as in figure 6 in a larger scale (0,105-0,130 seconds). Also here only small differences may be identified.

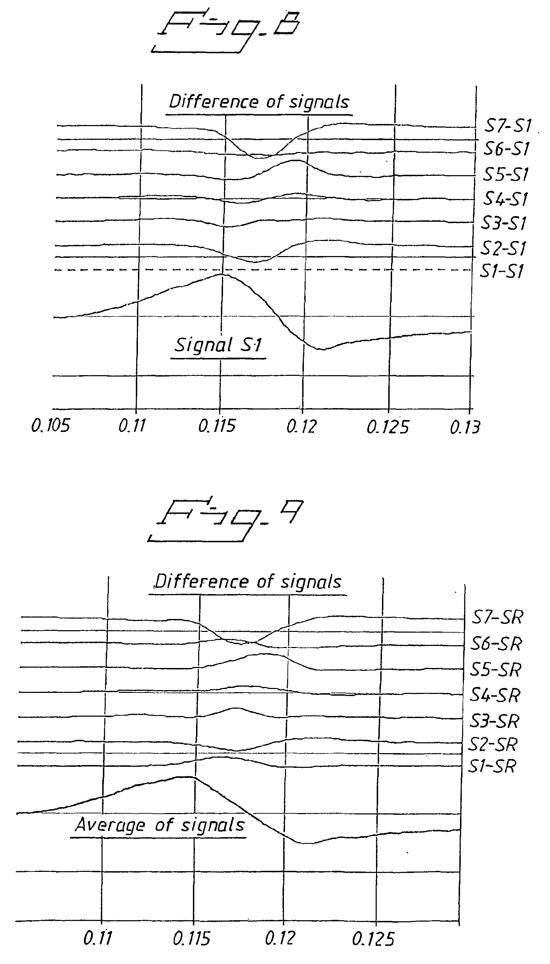

Figure 8 shows differences of signal recordings of seven dot electrodes (S1-S7) of a multi-dot electrode unit where electrode S 1 is used as a reference. The time scale is 0,105-0,130 seconds. The signal S6-S1 is cancelled out because the signals S6 and S1 are too similar. Investigations have shown as a rule that one or two of the bipolar signals are cancelled out. Which pair that is cancelled out cannot be predicted. Even if one pair is good and another is bad the situation may be the opposite due to varying conditions such as varying propagation vector or activity. This illustrates that the use of one bipolar signal from the multi-dot electrode unit is not sufficient and if more bipolar signals than one are used selection problems may occur.

Figure 9 shows differences of signal recordings of seven dot electrodes (S1-S7) of a multi-dot electrode unit where the synthetic reference signal (SR) calculated according to the present invention is used as a reference. The time scale is 0,105-0,130 seconds. The signals S1 to S7 are unipolar R-wave signals from the multi-dot electrode unit placed in the right ventricle of an animal using the indifferent electrode at the housing as reference. The signal at the bottom is the average of the signals, i.e. the synthetic reference signal.

These signal differences (S1-SR, ... ,S7-SR) illustrate that there is a low but significant signal amplitude in all signal recordings.

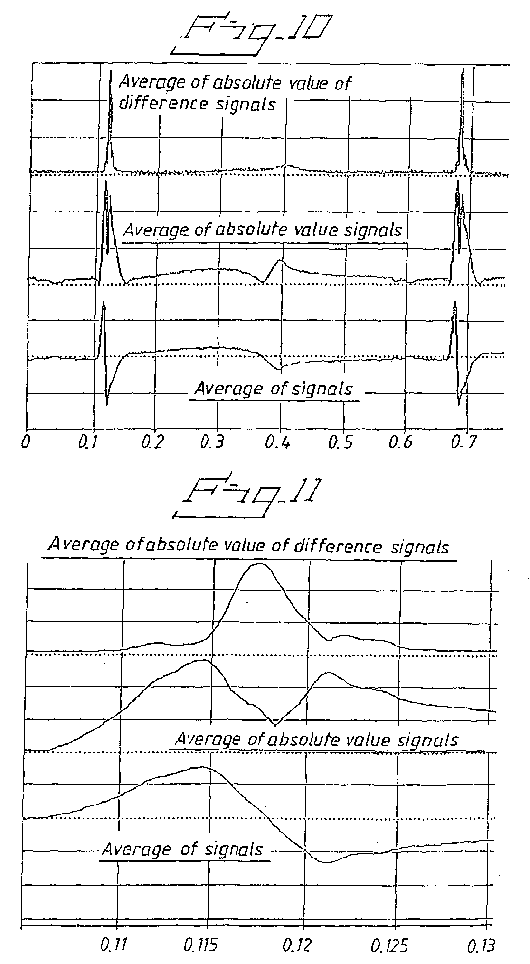

Figure 10 shows three signal tracings of different processed signals.

The signal at the top show the average of absolute value of difference signals processed according to the whole processing chain as earlier described according to the invention, i.e. all the signal differences created with use of the synthetic reference (S1-SR, ... ,S7-SR) are rectified and then added together. The heart activity is easy to detect, as the pulse amplitude is very distinguished. Detection occurs when the pulse amplitude is higher than a preset detection amplitude threshold (not shown).

The second signal (in the middle) shown in figure 10 is included to show that other signal processing also may be used to get a detectable signal result from all the electrode signals, in this case the average of the absolute amplitude values of all unipolar signals. However, the signal processing used according to the present invention achieves a more distinguished result, which has superior detection qualities.

The signal at the bottom of figure 10 is the average of the dot electrode potentials, i.e. the synthetic reference signal (SR).

Figure 11 shows the same signals as in figure 10 but with another time scale (0,105 to 0, 130 seconds). As in figure 10 the signal at the top in the figure illustrates the use of a signal processing according to the present invention. Investigations have shown that this signal shape has less variation than the variation in signal shape of each dot electrode signal, and also less variation than the variations of the average signal.

The present invention is not limited to the above-described preferred embodiments. Various alternatives, modifications and equivalents may be used. Therefore, the above embodiments should not be taken as limiting the scope of the invention, which is defined by the appending claims.

**Claims**

1. Medical system for detecting heart events including an electrode lead provided with a multi-dot electrode unit (4) that comprises at least three dot electrodes (8), said multi-dot electrode unit being adapted to be used for intracorporal sensing of heart signals, **characterized in that** heart signals sensed by each of the dot electrodes are applied to a processing means (18, 20, 22, 24) where the signals are combined and a synthetic reference (SR) signal is determined, the differences between each dot electrode heart signal and the synthetic reference voltage are determined, and an indication signal is formed based upon said differences, wherein said indication signal is used to detect heart events.

2. Medical system according to claim 1, **characterized in that** the synthetic reference signal (SR-signal) is determined according to the formula:

$$SR\text{-signal} = 1/N \ x \ \Sigma \ (U_1 + ... + U_N),$$

where

N is the number of dot electrodes, and $U_1 ... U_N$ are the dot electrode potentials in relation to an electrical reference point.

3. Medical system according to claim 2, **characterized in that** for each dot electrode a differential dot electrode value $A_{diff(i)}$ is determined by the formula:

$$A_{diff(i)} = U_i - SR\text{-signal},$$

where i = 1 ... N.

4. Medical system according to claim 3, **characterized in that** said indicating signal is formed by adding together the absolute values of $A_{diff(i)}$, where i = 1 ... N.

5. Medical system according to claim 3, **characterized in that** said indicating signal is formed by adding together the squared values of $A_{diff(i)}$, where i = 1 ... N.

6. Medical system according to claim 3, **characterized in that** said indicating signal is based upon the signal contents of $A_{diff(i)}$, where i = 1 ...N.

7. Medical system according to claim 1, **characterized in that** said indication signal is applied to a discrimination means adapted to generate an detection signal if the indication signal fulfils predetermined heart event detection criteria.

8. Medical system according to claim 1, **characterized in that** said processing means is arranged in said electrode lead in connection with the multi-dot electrode unit.

9. Medical system according to claim 1, **characterized in that** said multi-dot electrode unit is arranged at the distal end of said electrode lead.

10. Medical system according to claim 1, **characterized in that** said synthetic reference signal is determined as the average value of at least three of the detected dot electrode potentials.

**11.** Medical system according to claim 1, **characterized in that** said processing means is arranged in an implantable medical device and electrically connected to the multi-dot electrode unit via conducting means in said electrode lead.

**12.** Medical system according to claim 1, **characterized in that** multi-dot electrode unit may also be used to apply stimulation pulses to tissue.

**13.** Medical system according to claim 1, **characterized in that** said processing means is adapted to change mode of operation for the multi-dot electrode unit between a detection-mode and a stimulation-mode.

**Patentansprüche**

**1.** Medizinisches System zum Detektieren von Herzereignissen, enthaltend eine Elektrodenleitung, die mit einer Mehrpunkt-Elektrodeneinheit (4) ausgestattet ist, welche wenigstens drei Punkt-Elektroden (8) enthält, wobei die genannte Mehrpunkt-Elektrodeneinheit ausgelegt ist, zum Abfühlen von Herzsignalen innerhalb des Körpers benutzt zu werden, **dadurch gekennzeichnet, dass** die durch die jeweiligen Punkt-Elektroden abgefühlten Herzsignale einer Verarbeitungsvorrichtung (18, 20, 22, 24) zugeführt werden, wo die Signale kombiniert werden und ein synthetisches Referenzsignal (SR) bestimmt wird, die Differenzen zwischen jedem Punkt-Elektroden-Herzsignal und der synthetischen Referenzspannung bestimmt werden und ein Anzeigesignal auf der Grundlage der genannten Differenzen gebildet wird, wobei das Anzeigesignal zum Detektieren von Herzereignissen benutzt wird.

**2.** Medizinisches System nach Anspruch 1, **dadurch gekennzeichnet, dass** das synthetische Referenzsignal (SR-Signal) gemäß der Formel:

$$SR\text{-}Signal = 1/N \times \Sigma\, (U_1 + \ldots + U_N)$$

bestimmt wird, wobei

N die Anzahl der Punkt-Elektroden ist, und $U_1 \ldots U_N$ die Punkt-Elektrodenpotenziale bezüglich eines elektrischen Referenzpunktes sind.

**3.** Medizinisches System nach Anspruch 2, **dadurch gekennzeichnet, dass** für jede Punkt-Elektrode ein differentieller Punkt-Elektrodenwert ($A_{diff(i)}$ gemäß der Formel:

$$A_{diff(i)} = U_i - SR\text{-}Signal,$$

bestimmt wird, wobei i = 1 ... N.

**4.** Medizinisches System nach Anspruch 3, **dadurch gekennzeichnet, dass** das Anzeigesignal durch Addieren der absoluten Werte von $A_{diff(i)}$ gebildet wird, wobei i = 1 ... N.

**5.** Medizinisches System nach Anspruch 3, **dadurch gekennzeichnet, dass** das Anzeigesignal durch Addieren der Quadratwerte von $A_{diff(i)}$ gebildet wird, wobei i = 1 ... N.

**6.** Medizinisches System nach Anspruch 3, **dadurch gekennzeichnet, dass** das Anzeigesignal auf den Signalinhalten von $A_{diff(i)}$ beruht, wobei i = 1 ... N.

**7.** Medizinisches System nach Anspruch 1, **dadurch gekennzeichnet, dass** das Anzeigesignal einer Unterscheidungsvorrichtung zugeführt wird, die ausgelegt ist, ein Detektionssignal zu erzeugen, falls das Anzeigesignal vorbestimmte Herzereignisdetektionskriterien erfüllt.

**8.** Medizinisches System nach Anspruch 1, **dadurch gekennzeichnet, dass** die Verarbeitungsvorrichtung in der genannten Elektrodenleitung in Verbindung mit der Mehrpunkt-Elektrodeneinheit angeordnet ist.

**9.** Medizinisches System nach Anspruch 1, **dadurch gekennzeichnet, dass** die genannte Mehrpunkt-Elektrodeneinheit am distalen Ende der genannten Elektrodenleitung angeordnet ist.

**10.** Medizinisches System nach Anspruch 1, **dadurch gekennzeichnet, dass** das synthetische Referenzsignal als Mittelwert wenigstens dreier der detektierten Punkt-Elektrodenpotenziale bestimmt wird.

**11.** Medizinisches System nach Anspruch 1, **dadurch gekennzeichnet, dass** die Verarbeitungsvorrichtung in einer implantierbaren medizinischen Vorrichtung angeordnet und mit der Mehrpunkt-Elektrodeneinheit über Leiter in der Elektrodenleitung elektrisch verbunden ist.

**12.** Medizinisches System nach Anspruch 1, **dadurch gekennzeichnet, dass** die Mehrpunkt-Elektrodeneinheit auch benutzbar ist, dem Gewebe Stimulationsimpulse zuzuführen.

**13.** Medizinisches System nach Anspruch 1, **dadurch gekennzeichnet, dass** die Verarbeitungsvorrichtung ausgelegt ist, für die Mehrpunkt-Elektrodeneinheit den Betriebsmodus zwischen einem Detektionsmodus und einem Stimulationsmodus zu ändern.

**Revendications**

1.  Système médical pour détecter des évènements cardiaques, comprenant une dérivation d'électrode munie d'une unité (4) d'électrode multi-points qui comprend au moins trois électrodes (8) à point, l'unité d'électrode multi-points étant conçue pour être utilisée pour la détection intracorporelle de signaux cardiaques,
    **caractérisé en ce que** l'on applique des signaux cardiaques, détectés par chacune des électrodes à point, à un moyen (18, 20, 22, 24) de traitement, dans lequel les signaux sont combinés et un signal synthétique témoin (SR) est déterminé, les différences entre chaque signal cardiaque d'électrode à point et le signal synthétique témoin étant déterminées et un signal d'indication étant formé sur la base de ces différences, le signal d'indication étant utilisé pour détecter des évènements cardiaques.

2.  Système médical suivant la revendication 1, **caractérisé en ce que** l'on détermine le signal synthétique témoin (signal SR) par la formule

    $$\text{signal SR} = 1/N \times \Sigma (U_1 + \ldots + U_N),$$

    dans laquelle,

    N est le nombre d'électrodes à point, et $U_1 \ldots U_N$ sont des potentiels d'électrodes à point en relation à un point de référence électrique.

3.  Système médical suivant la revendication 2, **caractérisé en ce que** l'on détermine pour chaque électrode à point une valeur $A_{diff(i)}$ différentielle d'électrode à point par la formule :

    $$A_{diff(i)} = U_i - \text{signal SR},$$

    dans laquelle i = 1...N.

4.  Système médical suivant la revendication 3, **caractérisé en ce que** l'on forme le signal d'indication en ajoutant les valeurs absolues de $A_{diff(i)}$ dans laquelle i = 1... N.

5.  Système médical suivant la revendication 3, **caractérisé en ce que** l'on forme le signal d'indication en ajoutant les valeurs élevées au carré de

$A_{diff(i)}$, dans laquelle i=1....N.

6.  Système médical suivant la revendication 3, **caractérisé en ce que** l'on base le signal d'indication sur les contenus de signal de $A_{diff(i)}$, dans laquelle i = 1....N.

7.  Système médical suivant la revendication 1, **caractérisé en ce que** l'on applique le signal d'indication à un moyen de discrimination conçu pour produire un signal de détection, si le signal d'indication satisfait à un critère déterminé à l'avance de détection d'évènement cardiaque.

8.  Système médical suivant la revendication 1, **caractérisé en ce que** l'on met le moyen de traitement dans la dérivation d'électrode en liaison avec l'unité d'électrode multi-points.

9.  Système médical suivant la revendication 1, **caractérisé en ce que** l'on met l'unité d'électrode multi-points à l'extrémité distale de la dérivation d'électrode.

10. Système médical suivant la revendication 1, **caractérisé en ce que** l'on détermine le signal synthétique témoin comme la moyenne d'au moins trois des potentiels détectés d'électrodes à point.

11. Système médical suivant la revendication 1, **caractérisé en ce que** l'on met le moyen de traitement dans un dispositif médical implantable et on le relie électriquement à l'unité d'électrode multi-points par l'intermédiaire d'un moyen conducteur dans la dérivation d'électrode.

12. Système médical suivant la revendication 1, **caractérisé en ce que** l'on peut utiliser aussi l'unité d'électrode multi-points pour appliquer des impulsions de stimulation à du tissu.

13. Système médical suivant la revendication 1, **caractérisé en ce que** l'on adapte au moyen de traitement pour changer de mode de fonctionnement de l'unité d'électrode multi-points entre un mode de détection et un mode stimulation.

Fig. 1

Fig. 2

9

Fig.3

Synthetic Reference Voltage

$SR = 1/N \times \Sigma(U_i - U_G)$

$A_{diff(1)} = U_1 - SR$

$A_{diff(2)} = U_2 - SR$

$A_{diff(N)} = U_N - SR$

20

18

R/N

16

$U_1 - U_G$

$U_2 - U_G$

$U_N - U_G$

Dot 1

Dot 2

Dot N

Fig. 4

Fig. 5

## Fig. 6

Unipolar signals

0          0.5          1          1.5          2          2.5          3

## Fig. 7

Unipolar signals

S7

S6

S5

S4

S3

S2

S1

0.105          0.11          0.115          0.12          0.125          0.13

Fig. 8

Difference of signals

S7-S1
S6-S1
S5-S1
S4-S1
S3-S1
S2-S1
S1-S1

Signal S1

0.105    0.11    0.115    0.12    0.125    0.13

Fig. 9

Difference of signals

S7-SR
S6-SR
S5-SR
S4-SR
S3-SR
S2-SR
S1-SR

Average of signals

0.11    0.115    0.12    0.125

## Fig. 10

Average of absolute value of difference signals

Average of absolute value signals

Average of signals

0   0.1   0.2   0.3   0.4   0.5   0.6   0.7

## Fig. 11

Average of absolute value of difference signals

Average of absolute value signals

Average of signals

0.11   0.115   0.12   0.125   0.13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6064905 A

- US 4848352 A